# EUROPÄISCHE PATENTANMELDUNG

(11) **EP 1 857 140 A2**
(43) Veröffentlichungstag der Anmeldung: **21.11.2007**
(21) Anmeldenummer: 07008164.1
(22) Anmeldetag: 21.04.2007
(51) Int. Cl.: A61N 1/362, A61N 1/39

(54) **Herzstimulator**

(30) Priorität: 19.05.2006 DE 102006023517
(71) Anmelder: BIOTRONIK CRM Patent AG, 6341 Baar (CH)
(72) Erfinder: Dörr, Thomas, 12437 Berlin (DE); Becker, Frank, 10409 Berlin (DE); Radtke, Torsten, 12589 Berlin (DE)
(74) Vertreter: Lindner-Vogt, Karin L.

(57) **Zusammenfassung**

Die Erfindung betrifft einen Herzstimulator (10) mit einer Stimulationseinheit (1, 3, 5), verbunden mit einer Stimulationselektrode und einen Hochspannungskondensator (5) aufweisend, einem Detektor (2) zum Verarbeiten aufgenommener physiologischer Signale und zum Detektieren einer akuten ventrikulären Tachykardie, und einer Steuereinheit (4), verbunden mit dem Detektor (2) und der Stimulationseinheit (1, 3, 5) und ausgebildet, die Stimulationseinheit (1, 3, 5) zur Abgabe von Stimulationsimpulsen anzusteuern, wobei das Ausgangssignal des Detektors (2) bei stabilem Herzrhythmus mit einer Frequenz oberhalb einer vorgegebenen Tachykardiedetektionsgrenze ein Tachykardiesignal ist, die Steuereinheit (4) ausgebildet ist, auf ein Tachykardiesignal hin die Stimulationseinheit (1, 3, 5) zur Abgabe einer eine antitachykarde Therapie bildenden Folge von Stimulationsimpulsen anzusteuern und danach das Laden des Hochspannungskondensators (5) auszulösen, und der Detektor (2) ausgebildet ist, während des Ladens aufgenommene Signale zu verarbeiten und bei einer Frequenz unterhalb einer vorgegebenen Tachykardieredetektionsgrenze ein Tachykardieende-Signal zu erzeugen, wobei die Steuereinheit (4) auf ein Tachykardieende-Signal hin das Laden des Hochspannungskondensators (5) sofort abbricht.

## Beschreibung

Die Erfindung betrifft einen Herzstimulator mit einer Stimulationseinheit, die mit einer Stimulationselektrode zum Stimulieren eines Ventrikels eines Herzens verbunden ist oder verbunden werden kann. Die Stimulationseinheit ist ausgebildet, sowohl Stimulationsimpulse als auch Defibrillationsschocks zu erzeugen und weist hierzu zumindest einen Hochspannungskondensator auf, in dem die für einen Defibrillationsschock nötige elektrische Energie zu speichern ist. Außerdem weist der Herzstimulator einen Detektor auf, der dazu ausgebildet ist, vom Herzen aufgenommene physiologische Signale zu verarbeiten und auf Basis dessen das Vorliegen einer akuten ventrikulären Tachykardie oder Fibrillation zu detektieren. Weiterer Bestandteil des Herzstimulators ist eine Steuereinheit, die mit dem Detektor und der Stimulationseinheit verbunden ist und die ausgebildet ist, auf ein Ausgangssignal des Detektors anzusprechen und die Stimulationseinheit entweder zur Abgabe einer eine antitachykarde Therapie bildenden Folge von Stimulationsimpulsen oder eines Defibrillationsschocks anzusteuern.

Derartige Herzstimulatoren sind grundsätzlich bekannt und werden auch als implantierbare Kardioverter Defibrillatoren (ICD) bezeichnet. Es handelt sich also bei den hier gemeinten Herzstimulatoren in erster Linie um implantierbare Herztherapiegeräte, die in der Lage sind, eine Tachykardie eines Herzens zu therapieren.

Mit Tachykardien sollen im Rahmen dieser Anmeldung sowohl Tachykardien im engeren Sinne, die sich durch einen stabilen Herzrhythmus mit pathologisch hoher Frequenz auszeichnen, als auch Fibrillationen gemeint sein. Bekannte Therapien, die durch einen Herzstimulator der eingangs genannten Art abgegeben werden können, sind eine antitachykarde Stimulation oder ein Defibrillationsschock.

Ein Defibrillationsschock ist ein elektrischer Stromstoß, der in das Herz abgegeben wird und der eine ausreichend hohe Spannung und Energie besitzt, um eine von Fibrillation betroffene Herzkammer vollständig zu erregen und damit refraktär zu machen. Auf diese Weise werden für Fibrillationen typische kreisende Erregungen unterbrochen. Im Falle einer Tachykardie im engeren Sinne, die - soweit der Ventrikel betroffen ist - häufig auch als ventrikuläre Tachykardie bezeichnet und mit VT abgekürzt wird (im Unterschied zur ventrikulären Fibrillation VF) ist häufig eine erfolgreiche Therapie auf dem Wege der antitachykarden Stimulation (anti tachyarrhythmia pacing; ATP) möglich. Im Rahmen der antitachykarden Stimulation gibt der Herzstimulator eine Folge von Stimulationsimpulsen aus, deren Energie wesentlich geringer ist, als die Energie eines Defibrillationsschocks und die daher auch nicht schmerzhaft sind. Im Rahmen der antitachykarden Stimulation werden solche Stimulationsimpulse vergleichsweise niedriger Energie mit einer Frequenz abgegeben, die größer ist, als die Frequenz der erfassten Tachykardie. In vielen Fällen kann auf diese Weise eine Tachykardie beendet werden, ohne dass der Patient Schmerzen erleiden muss oder dass der Energiebedarf besonders hoch ist.

Da der Erfolg einer antitachykarden Stimulation nicht immer gegeben ist, kann es im Nachgang einer erfolglosen antitachykarden Stimulation erforderlich sein, einen Defibrillationsschock abzugeben.

Aus US 6,718,204 ist es beispielsweise bekannt, eine derartige antitachykarde Stimulation nach Erfassen einer Tachykardie abzugeben, während oder kurz bevor ein Hochspannungskondensator aufgeladen wird, um nötigenfalls einen Defibrillationsschock im Anschluss an die tachykarde Stimulation abgeben zu können.

Dabei bestehen die widersprüchlichen Anforderungen, einerseits möglichst Energie einsparen zu wollen, also das Laden des Hochspannungskondensators möglichst zu vermeiden. Andererseits soll ein Defibrillationsschock nach erfolgloser antitachykarder Stimulation so schnell wie möglich abgegeben werden können, was grundsätzlich einen bereits geladenen Kondensator voraussetzt.

Der Erfindung liegt die Aufgabe zugrunde, eine möglichst optimale Lösung für dieses Dilemma zu finden.

Erfindungsgemäß besteht die Lösung der Aufgabe darin, dass ein - vorzugsweise implantierbarer - Herzstimulator der eingangs genannten Art dazu ausgebildet ist, nach Erfassen einer schnellen monomorphen ventrikulären Tachykardie mit einer Frequenz oberhalb einer Fibrillationsdetektionsgrenze zunächst eine antitachykarde Stimulation auszulösen, um sofort nach Ende der Folge von Stimulationsimpulsen, die die antitachykarde Stimulationstherapie bilden, das Laden des Hochspannungskondensators auszulösen und während des Ladens des Hochspannungskondensators durch Auswerten von im Herzen aufgenommenen physiologischen Signalen zu bestimmen, ob die antitachykarde Stimulationstherapie erfolgreich war oder nicht. Sollte die Auswertung ergeben, dass die antitachykarde Stimulationstherapie erfolgreich war, wird das Laden des Hochspannungskondensators sofort abgebrochen, so dass nicht die gesamte Energie zum vollständigen Laden des Kondensators verloren geht. Sollte hingegen die Auswertung der nach Abgabe der antitachykarden Stimulationstherapie aufgenommenen physiologischen Signale ergeben, dass die zu behandelnde Tachykardie anhält, wird die Ladung des Hochspannungskondensators fortgesetzt und schließlich ein Defibrillationsschock abgegeben. Dies bedeutet, dass das Laden des Hochspannungskondensators nicht erst begonnen wird, nachdem die Auswertung des im Herzen aufgenommenen physiologischen Signals ergeben hat, dass die Tachykardie fortbesteht. Dies erlaubt es, den Defibrillationsschock so schnell wie möglich abzugeben.

Der Herzstimulator ist vorzugsweise dazu ausgebildet, wenigstens zwei Arten von Tachykardien zu detektieren und an die Detektion der jeweiligen Art von Tachykardie unterschiedliche Folgen zu knüpfen. Im Falle eines derartigem bevorzugten Herzstimulators führt nur die Detektion einer stabilen Tachykardie mit einer Frequenz in einem Frequenzbereich, der einer Fibrillation entspricht, dazu, dass die Steuereinheit zunächst eine antitachykarde Stimulation auslöst, um nach deren Abschluss gleichzeitig mit dem Beginn (und nicht erst bei Vorliegen des Ergebnisses) der Erfolgskontrolle das Laden des Hochspannungskondensators für die Defibrillation auszulösen. Diese Therapiefolge wird durch ein Dignal ausgelöst, dass hier als Tachykardiesignal bezeichnet wird. Im Falle einer Tachykardie mit niedrigerer Frequenz (unterhalb der Fibrillations-Detektionsgrenze) erzeugt der Detektor ein anderes Signal, dass die Steuereinheit dazu veranlasst, eine antitachykarde Stimulation auslösen, aber nach deren Ende nicht das Laden des Hochspannungskondensators für die Defibrillation.

In diesem Sinne spricht der Detektor auf Herzfrequenzen zwischen 150 und 300 Schlägen pro Minute als Fibrillations-Detektionsgrenze (entsprechend Herzintervallen zwischen 400 ms und 200 ms) derart an, dass die Steuereinheit die erfindungsgemäße Folge von antitachykarder Stimulation und anschließendem, sofortigen Laden des Hochspannungskondensators auslöst, während der Detektor auf Herzfrequenzen zwischen 100 und 150 Schlägen pro Minute bzw. Herzintervallen zwischen 600 ms und 400 ms, derart anspricht, dass er die Steuereinheit dazu veranlasst, lediglich eine antitachykarde Stimulation, aber kein anschließendes Laden des Hochspannungskondensators auszulösen.

Dabei ist es vorteilhaft, wenn der Detektor wenigstens für den ersten der beiden vorgenannten Fälle dazu ausgebildet ist, die Stabilität des tachykarden Herzrhythmus anhand eines x aus y Kriteriums in dem Sinne zu erfassen, dass eine stabile Tachyarrhythmie detektiert wird, wenn wenigstens X ventrikuläre Intervalle innerhalb der letzten Y aufeinanderfolgenden ventrikulären Intervalle kürzer als die vorgegebene Fibrillationsgrenze sind. Geeignete Werte für X sind Werte zwischen 6 und 30. Geeignete Werte für Y sind Werte zwischen 8 und 31.

Erfindungsgemäß ist der Detektor des Herzstimulators ausgebildet, einen stabilen Herzrhythmus mit einer Frequenz oberhalb einer vorgegebenen Fibrillations-Detektionsgrenze zu erkennen und daraufhin ein Ausgangssignal zu erzeugen, das die Steuereinheit dazu veranlasst, die Stimulationseinheit zur Abgabe einer eine antitachykarde Therapie bildenden Folge von Stimulationsimpulsen anzusteuern. Die Steuereinheit ist dazu ausgebildet, unmittelbar im Anschluss an die Folge der die antitachykarde Therapie bildenden Folge von Stimulationsimpulsen das Laden des Hochspannungskondensators auszulösen, während der Detektor gleichzeitig - also ebenfalls unmittelbar nach Abgabe der eine antitachykarde Therapie bildenden Folge von Stimulationsimpulsen - vom Herzen aufgenommene physiologische Signale daraufhin analysiert, ob die antitachykarde Therapie erfolgreich war oder nicht. Erzeugt der Detektor daraufhin ein Ausgangssignal, das eine erfolgreiche antitachykarde Therapie kennzeichnet, spricht die Steuereinheit auf dieses Signal an und bricht das Laden des Hochspannungskondensators sofort ab. Die Detektionsgrenze, auf die hin der Detektor ein Ausgangssignal abgibt, das die Steuereinheit veranlasst, die antitachykarde Therapie auszulösen, kann als Tachykardiedetektionsgrenze oder als Fibrillationsdetektionsgrenze bezeichnet werden und entspricht einer Herzfrequenz, die üblicherweise einer Fibrillation zugerechnet wird. Neben dem Frequenzkriterium wendet der Detektor auch ein Stabilitätskriterium bei der Auswertung der vom Herzen stammenden physiologischen Signale an und erzeugt das Ausgangssignal, das die Steuereinheit dazu veranlasst, eine antitachykarde Therapie auszulösen, nur dann, wenn der Herzrhythmus stabil ist. Das vom Detektor abgegebene Steuersignal wird hier als Tachykardiesignal bezeichnet. Dieses Tachykardiesignal kann unter Umständen ein anderes Signal sein, als das Ausgangssignal, das der Detektor erzeugt, wenn er eine erhöhte, stabile Herzfrequenz erfasst, die zwar einer Tachykardie zuzurechnen ist, die aber noch nicht die Frequenzgrenze zur Fibrillation überschritten hat.

Das Ausgangssignal, das der Detektor nach Erfassen einer erfolgreichen antitachykarden Therapie erzeugt und das die Steuereinheit veranlasst, das Laden des Hochspannungskondensators abzubrechen, wird für die Zwecke dieser Anmeldung als Tachykardieende-Signal bezeichnet.

Dieses Tachykardieende-Signal erzeugt der Detektor gemäß einer bevorzugten Ausführungsvariante der Erfindung dann, wenn nach Abschluss der antitachykarden Stimulation vorzugsweise 3 von 4 aufeinanderfolgende, erfasste ventrikuläre Intervalle länger als eine vorgegebene Tachykardie-Detektionsgrenze sind. Ein bevorzugter Wert für die Tachykardie-Detektionsgrenze sind 600 ms.

Gemäß dieser bevorzugten Ausführungsvariante gibt der Herzstimulator einen Defibrillationsschock ab, sofern die Steuereinheit bis zum Ende des Ladens des Hochspannungskondensators kein Tachykardieende-Signal seitens des Detektors erhalten hat. Die Abgabe des Defibrillationschocks erfolgt somit unmittelbar nach dem Abschluss des Ladens des Hochspannungskondensators, sofern das Laden des Hochspannungskondensators nicht zuvor auf ein Tachykardieende-Signal hin abgebrochen wird.

In einer alternativen Ausführungsvariante ist der Detektor ausgebildet, im Falle der Detektion eines mangelnden Therapieerfolges nach antitachykarder Therapie ein Tachykardieredetektionssignal zu erzeugen, das die Steuereinheit dazu veranlasst, das Laden des Hochspannungskondensators fortzusetzen und schließlich die Abgabe des Defibrillationsschocks auszulösen.

Für die Auswertung der vom Herzen stammenden physiologischen Signale nach Abgabe der antitachykarden Therapie vergleicht der Detektor die dann erfasste Herzfrequenz mit einem durch eine Tachykardieredetektionsgrenze vorgegebenen Frequenzwert. Dieser Frequenzwert entspricht vorzugsweise der Tachykardie - bzw. Fibrillationsdetektionsgrenze.

Das vom Detektor auszuwertende, vom Herzen stammende physiologische Signal ist vorzugsweise ein intrakardiales Elektrokardiogramm. Zur Aufnahme eines solchen intrakardialen Elektrokardiogramms ist der Detektor vorzugsweise mit einer Sensing-Elektrode verbunden oder kann mit einer solchen Sensing-Elektrode verbunden werden. Eine geeignete Sensing-Elektrode kann dabei auch eine Stimulationselektrode sein.

Vorzugsweise ist die Stimulationseinheit des Herzstimulators mit mindestens zwei unterschiedlichen Arten ventrikulärer Elektroden verbunden, nämlich mit wenigstens einer ventrikulären Stimulationselektrode sowie mit wenigstens einer ventrikulären Defibrillationselektrode, die eine größere Oberfläche aufweist, als die Stimulationselektrode. Vorzugsweise ist die Stimulationselektrode eine Tip- oder Ringelektrode, während die Defibrillationselektrode vorzugsweise eine von einem distalen Elektrodenleitungsende etwas entfernt angeordnete Wendelelektrode ist.

Die antitachykarde Stimulation kann in bevorzugten Ausführungsvarianten folgende Eigenschaften haben. Die Anzahl der die antitachykarde Therapie bildenden Folge von Stimulationsimpulsen ist vorzugsweise ein vorgegebener Wert zwischen 1 und 10. Das Intervall zwischen einzelnen Stimuli der Folge von Stimulationsimpulsen sowie zwischen dem zuletzt erfassten ventrikulären Ereignis und dem ersten Stimulus der Folge von Stimulationsimpulsen ist vorzugsweise auf eine Dauer von 75% bis 90% entweder des zuletzt erfassten ventrikulären Intervalls oder der durchschnittlichen Intervalldauer zwischen einer vorgegebenen Anzahl zu letzt erfasster, aufeinander folgender ventrikulärer Ereignisse eingestellt. Alternativ kann die Folge von Stimulationsimpulsen eine ansteigende Rate aufweisen, bei der ein Intervall zwischen zwei aufeinander folgenden Stimuli von anfänglich 350 ms bis 200 ms von Intervall zu Intervall jeweils um einen Wert zwischen 40 ms und 5 ms verkürzt wird.

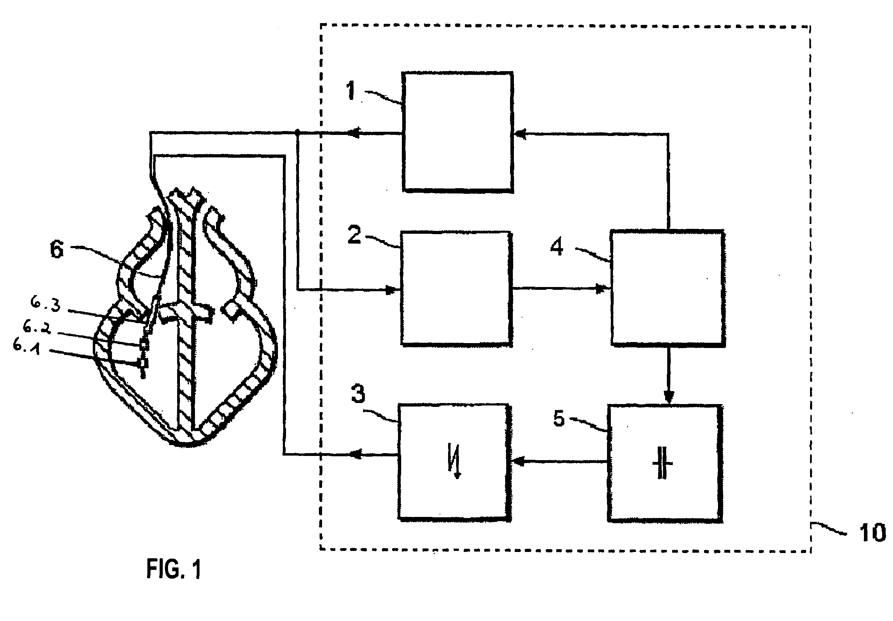

Die Erfindung soll nun anhand eines Ausführungsbeispiels mit Bezug auf die Figur 1 näher erläutert werden.

Hierzu zeigt die Figur 1 einen implantierbaren Herzstimulator 10, der über eine Elektrodenleitung 6 mit dem rechten Ventrikel eines Herzens in Verbindung steht. Die Elektrodenleitung 6 trägt eine ventrikuläre Tippelektrode 6.1 und eine ventrikuläre Ringelektrode 6.2 sowie eine Schockwendel 6.3 zur Abgabe eines Defibrillationsschocks. Die ventrikulären Tip- und Ringelektroden 6.1 und 6.2 dienen als Sensing-Elektroden. Die ventrikuläre Tippelektrode 6.1 dient darüber hinaus als Stimulationselektrode. Die ventrikuläre Tippelektrode 6.1 und die ventrikuläre Ringelektrode 6.2 sind über die Elektrodenleitung 6 mit einem Detektor 2 verbunden, der über die Elektroden 6.1 und 6.2 das aufgenommene elektrische Potential über entsprechende Eingangsverstärker verstärkt und so ein intrakardiales Elektrokardiogramm erzeugt. Der Detektor 2 ist ausgebildet, aus diesem intrakardialen Elektrokardiogrammsignal jeweils eine aktuelle Herzfrequenz abzuleiten und diese mit vorgegebenen Frequenzgrenzen zu vergleichen. Eine dieser vorgegebenen Frequenzgrenzen wird im Rahmen dieser Anmeldung mit Tachykardiedetektionsgrenze bzw. Fibrillationsgrenze bezeichnet und entspricht einer Herzfrequenz, ab der üblicherweise von einer Fibrillation des Herzens gesprochen wird. Darüber hinaus ist der Detektor 2 ausgebildet, die Stabilität der erfassten Herzfrequenz zu analysieren und ein Tachykardiedetektionssignal nur dann zu erzeugen, wenn die Auswertung des intrakardialen Elektrokardiogramms ergibt, dass die akute Herzfrequenz die Tachykardiedetektionsgrenze überschreitet und darüber hinaus so wenig schwankt, dass sie ein vorgegebenes Stabilitätskriterium erfüllt.

Der Detektor 2 ist mit einer Steuereinheit 4 verbunden, die auf ein jeweiliges Ausgangssignal des Detektors 2 anspricht.

Die Steuereinheit 4 ist ihrerseits ausgangsseitig mit einer Stimulationseinheit verbunden, die zum einen eine niedrige Energiestimulationseinheit 1 umfasst und zum anderen eine Defibrillationseinheit mit einem Hochspannungskondensator 5 und einer Schocksynchronisationseinheit 3.

Die Niedrigenergiestimulationseinheit 1 ist mit der ventrikulären Tippelektrode 6.1 verbunden und dazu ausgebildet, Stimulationsimpulse niedriger Energie und einer maximalen Spannung von weniger als 10 Volt über die ventrikuläre Tippelektrode 6.1 an ein Herz abzugeben. Die Niedrigenergiestimulationseinheit 1 ist insbesondere dazu ausgebildet, angesteuert von der Steuereinheit 4, eine schnelle Folge von Stimulationsimpulsen als antitachykarde Therapie mit einer Folgefrequenz der Stimulationsimpulse abzugeben, die über der vom Detektor 2 erfassten akuten Herzfrequenz liegt. Eine solche Folge von Stimulationsimpulsen gibt die Niedrigenergiestimulationseinheit 1 dann ab, wenn der Detektor nach Erfassen der zuvor erläuterten Bedingungen ein Tachykardiedetektionssignal erzeugt und an die Steuereinheit 4 abgegeben hat. Die Steuereinheit 4 ist dazu ausgebildet, unmittelbar nach Ende einer antitachykarden Therapie bildenden Folge von Stimulationsimpulsen das Laden des Hochspannungskondensators 5 auszulösen.

Der Detektor 2 ist ausgebildet, dass unmittelbar nach Ende der antitachykarden Therapie erfasste intrakardiale Elektrokardiogramm dahingehend auszuwerten, ob die antitachykarde Therapie erfolgreich war und die Tachykardie somit beendet wurde. Dazu vergleicht der Detektor eine aus dem intrakardialen Elektrokardiogramm nach Abgabe der antitachykarden Therapie abgeleitete Herzfrequenz mit einem Frequenzgrenzwert, der hier als Tachykardiedetektionsgrenze bezeichnet wird und einem Wert entspricht, der die Grenze zwischen einem nicht behandlungsbedürftigen schnellen Herzrhythmus und einer niederfrequenten Tachykardie mit einer Herzzykluslänge von 600 ms und weniger markiert. Diese Tachykardiedetektionsgrenze ist dementsprechend im Ausführungsbeispiel 100 Schläge pro Minute. Ist die nach Abgabe der antitachykarden Therapie erfasste Herzfrequenz geringer als die Tachykardiedetektionsgrenze, erzeugt der Detektor 2 ein Tachykardieende-Signal.

Auf dieses Tachykardieende-Signal spricht die Steuereinheit 4 an und bricht das bereits begonnene Laden des Hochspannungskondensators 5 sofort ab. Falls bis zum vollständigen Aufladen des Hochspannungskondensators, d.h. bis zum Ladeende, kein Tachykardieende detektiert und somit auch kein Tachykardieende-Signal generiert wird, wird das Laden des Hochspannungskondensators 5 bis zu Ladeende fortgesetzt und die Schock-Synchronisationseinheit 3 dazu veranlasst, den Defibrillationsschock wenn möglich synchronisiert abzugeben. Die Synchronisation des Defibrillationsschocks erfolgt vorzugsweise auf ein ventrikuläres Herzsignal (R-Zacke) oder auf einen ventrikulären Stimulus. Nur wenn (bei einem Schock ohne Bestätigung) innerhalb von 2s nach Ladeende des Hochspannungskondensators kein ventrikuläres Ereignis auftritt, wird der Schock unsynchronisiert abgegeben.

Alternativ könnte die Steuereinheit dazu ausgebildet sein, die Abgabe des Defibrillationschock nach Ladeende zusätzlich davon abhängig zu machen, dass die dann aktuelle Herzfrequenz oberhalb einer Tachykardieredetektionsgrenze liegt. Ergibt in diesem Fall die Auswertung des nach Ladeende des Hochspannungskondensators erfassten intrakardialen Elektrokardiogramms durch den Detektor 2, dass die Herzfrequenz oberhalb der Tachykardieredetektionsgrenze liegt, erzeugt der Detektor 2 ein Tachykardieredetektionssignal, welches die Steuereinheit 4 dazu veranlasst, die Schock-Synchronisationseinheit 3 so anzusteuern, dass diese einen nach Möglichkeit synchronisierten Defibrillationsschock abgibt.

Auf diese Weise ist der Herzstimulator 10 in der Lage, eine monomorphe ventrikuläre Tachykardie hoher Frequenz nach Möglichkeit mit einer antitachykarden Therapie zu beenden und im Falle eines mangelnden Erfolges der antitachykarden Therapie so schnell wie möglich einen Defibrillationsschock auszulösen, ohne dass im Falle einer erfolgreichen antitachykarden Therapie allzu viel Energie für das Laden eines Hochspannungskondensators verloren geht.

Über die vorstehend beschriebenen Merkmale hinaus kann der Herzschrittmacher alle üblichen Merkmale bekannter implantierbarer Herzschrittmacher bzw. Kardioverter/Defibrillatoren aufweisen, wie beispielsweise eine Batterie, eine Telemetrieeinheit zur Übertragung von Daten an ein Service-Center oder an ein externes Gerät wie ein Patientengerät, eine atriale Stimulationseinheit, eine atriale Defibrillationseinheit, eine atriale Sensingeinheit, ein linksventrikuläre Stimulations- und/oder Sensingeinheit usw..

## Patentansprüche

1. Herzstimulator (10) mit
einer Stimulationseinheit (1, 3, 5),
die mit einer Stimulationselektrode zum Stimulieren eines Ventrikels eines Herzens verbunden oder zu verbinden ist und
die ausgebildet ist, Stimulationsimpulse und Defibrillationsschocks zu erzeugen und
die zumindest einen Hochspannungskondensator (5) für das Speichern elektrischer Energie für einen Defibrillationsschock aufweist,
einem Detektor (2),
der ausgebildet ist, vom Herzen aufgenommene physiologische Signale zu verarbeiten und das Vorliegen einer akuten ventrikulären Tachykardie oder Fibrillation zu detektieren, und
einer Steuereinheit (4),
die mit dem Detektor (2) und der Stimulationseinheit (1, 3, 5) verbunden ist und
ausgebildet ist, auf ein Ausgangssignal des Detektors (2) anzusprechen und die Stimulationseinheit (1, 3, 5) zur Abgabe einer eine antitachykarde Therapie bildenden Folge von Stimulationsimpulsen oder eines Defibrillationsschocks anzusteuern.
**dadurch gekennzeichnet,**
**dass** das Ausgangssignal des Detektors (2) im Falle eines einen stabilen Herzrhythmus mit einer Frequenz oberhalb einer vorgegebenen Fibrillations-Detektionsgrenze wiedergegebenen physiologischen Signals ein Tachykardiesignal ist,
**dass** die Steuereinheit (4) ausgebildet ist, auf ein Tachykardiesignal des Detektors (2) hin die Stimulationseinheit (1, 3, 5) zur Abgabe einer eine antitachykarde Therapie bildenden Folge von Stimulationsimpulsen anzusteuern und im direkten Anschluss an die Abgabe der eine antitachykarde Therapie bildenden Folge von Stimulationsimpulsen das Laden des Hochspannungskondensators (5) auszulösen, und
**dass** der Detektor (2) ausgebildet ist, während des Ladens des Hochspannungskondensators (5) vom Herzen aufgenommene physiologische Signale zu verarbeiten und anhand wenigstens eines vorgegebenen Kriteriums auf das Vorliegen oder Nicht-Vorliegen einer Tachykardie nach Ende der eine antitachykarde Therapie bildenden Folge von Stimulationsimpulsen hin zu untersuchen und ein Tachykardieende-Signal zu erzeugen, falls das vom Herzen aufgenommene physiologische Signal auf Basis des vorgegebenen Kriteriums ein Nicht-Vorliegen einer Tachykardie anzeigt,
wobei die Steuereinheit (4) ausgebildet ist, auf ein Tachykardieende-Signal hin das Laden des Hochspannungskondensators (5) sofort abzubrechen.

2. Herzstimulator nach Anspruch 1, **dadurch gekennzeichnet, dass** das vorgegebene Kriterium ein Tachykardieende-Kriterium ist, welches ein vom Herzen aufgenommene physiologische Signale bei Nichtvorliegen einer Tachykardie kennzeichnet und dass der Detektor ausgebildet ist, das Tachykardieende-Signal zu erzeugen, falls das Tachykardieende-Kriterium erfüllt.

3. Herzstimulator nach Anspruch 2, **dadurch gekennzeichnet, dass** das Tachykardieende-Kriterium erfüllt ist, wenn das vom Herzen aufgenommene physiologische Signal anzeigt, dass eine vorgegebene Anzahl aufeinander folgender ventrikulärer Intervalle länger als eine vorgegebene Tachykardie-Detektionsgrenze.

4. Herzstimulator nach Anspruch 3, **dadurch gekennzeichnet, dass** die vorgegebene Tachykardie-Detektionsgrenze einen Wert zwischen 400 ms und 600 ms hat.

5. Herzstimulator nach Anspruch 3 oder 4, **dadurch gekennzeichnet, dass** die vorgegebene Anzahl ventrikulärer Intervalle 3 Intervalle innerhalb von 4 aufeinander folgender Intervalle ist.

6. Herzstimulator nach einem der Ansprüche 1 bis 5, **dadurch gekennzeichnet, dass** die Steuereinheit (4) ausgebildet ist, die Abgabe eines Defibrillationsschocks auszulösen, falls sie bis zum Ende der Ladezeit des Hochspannungskondensators kein Tachykardieende-Signal empfangen hat.

7. Herzstimulator nach Anspruch 1, **dadurch gekennzeichnet, dass** das vorgegebene Kriterium ein Tachykardieredetektions-Kriterium ist, welches ein vom Herzen aufgenommene physiologische Signale bei Vorliegen einer Tachykardie kennzeichnet und dass der Detektor ausgebildet ist, das Tachykardieende-Signal zu erzeugen falls das Tachykardieredetektions-Kriterium nicht erfüllt ist.

8. Herzstimulator nach Anspruch 7, **dadurch gekennzeichnet, dass** der Detektor (2) ausgebildet ist, während des Ladens des Hochspannungskondensators (5) vom Herzen aufgenommene physiologische Signale zu verarbeiten und im Falle eines einen Herzrhythmus mit einer Frequenz oberhalb einer vorgegebenen Tachykardieredetektionsgrenze wiedergebenden physiologischen Signals ein Tachykardieredetektions-Signal zu erzeugen und die Steuereinheit (4) ausgebildet ist, auf ein Tachykardieredetektions-Signal hin die Stimulationseinheit (1, 3, 5) zur Abgabe eines Defibrillationsschocks anzusteuern.

9. Herzstimulator nach Anspruch 7 oder 8, **dadurch gekennzeichnet, dass** die durch die Tachykardie-Detektionsgrenze und die Tachykardieredetektionsgrenze vorgegebenen Herzfrequenzen identisch sind.

10. Herzstimulator nach einem der Ansprüche 1 bis 9, **dadurch gekennzeichnet, dass** die Fibrillations-Detektionsgrenze einen Wert zwischen 150/min und 300/min hat.

11. Herzstimulator nach einem der Ansprüche 1 bis 10, **dadurch gekennzeichnet, dass** der Detektor ausgebildet ist, das Tachykardiesignal zu erzeugen, wenn die Herzfrequenz des vom Herzen aufgenommenen physiologischen Signals in einer Anzahl von X Herzzyklen aus einer vorgegebenen Anzahl von Y aufeinander folgenden Herzzyklen eine Herzfrequenz oberhalb der Fibrillations-Detektionsgrenze aufweist, wobei X kleiner oder gleich Y ist und X einen Wert zwischen 6 und 30 und Y einen Wert zwischen 8 und 31 hat.

12. Herzstimulator nach einem der Ansprüche 1 bis 11, **dadurch gekennzeichnet, dass** die Stimulationseinheit eine Niedrigenergiestimulationseinheit (1) umfasst, die zur Abgabe von Stimulationsimpulsen ausgebildet ist, sowie eine Defibrillationseinheit, die den Hochspannungskondensator (5) und eine Defibrillationsschock-Synchronisiereinheit (3) umfasst und zur Abgabe eines Defibrillationsschocks ausgebildet ist.

13. Herzstimulator nach Anspruch 12, **dadurch gekennzeichnet, dass** die Niedrigenergiestimulationseinheit (1) mit einer ventrikulären TippElektrode (6.1) verbunden ist oder zu verbinden ist.

14. Herzstimulator nach Anspruch 12 oder 13, **dadurch gekennzeichnet, dass** die Defibrillationseinheit mit einer wendelförmigen Defibrillationselektrode (6.3) verbunden ist oder zu verbinden ist.

15. Herzstimulator nach einem der Ansprüche 1 bis 14, **dadurch gekennzeichnet, dass** der Detektor (2) eine Sensing-Einheit mit einem Eingangsverstärker zum Verstärken von im Herzen aufgenommener elektrischer Potentiale aufweist.

16. Herzstimulator nach einem der Ansprüche 1 bis 15, **dadurch gekennzeichnet, dass** der Detektor (2) mit einer ventrikulären Ringelektrode (6.2) und einer ventrikulären Tippelektrode (6.1) verbunden ist oder zu verbinden ist.

17. Herzstimulator nach einem der Ansprüche 1 bis 16, **dadurch gekennzeichnet, dass** der Detektor (2) Mittel zum Ableiten einer Herzfrequenz aus einem intrakardialen Elektrokardiogrammsignal sowie einem Komparator zum Vergleichen der abgeleiteten Herzfrequenz mit Frequenzgrenzwerten aufweist, von denen ein Herzfrequenzgrenzwert als Tachykardie-Detektionsgrenze vorgegeben ist.

18. Herzstimulator nach einem der Ansprüche 1 bis 17, **dadurch gekennzeichnet, dass** der Detektor (2) ausgebildet ist, aus einem intrakardialen Elektrokardiogrammsignal die Stabilität einer Herzfrequenz über mehrere Herzzyklen abzuleiten und mit einem Stabilitätskriterium zu vergleichen.
